# EUROPEAN PATENT SPECIFICATION

(11) **EP 1 987 121 B1**
(45) Date of publication and mention of the grant of the patent: **13.03.2013**
(21) Application number: 07712017.8
(22) Date of filing: 12.01.2007
(51) Int. Cl.: C11D 3/39, A01N 59/00, A01N 57/12, C11D 3/48

(54) **ENHANCED ACTIVITY BIOCIDAL HYROGEN PEROXIDE COMPOSITION**
BIOZIDE WASSERSTOFFPEROXIDZUSAMMENSETZUNG ERHÖHTER AKTIVITÄT
COMPOSITION DE PEROXYDE D HYDROGENE A MEILLEURE ACTIVITE BIOCIDE

(30) Priority: 13.01.2006 EP 06100346; 06.07.2006 EP 06116746
(43) Date of publication of application: 05.11.2008
(73) Proprietor: Aseptix Research B.V., 3632 AX Loenen Aan De Vecht (NL)
(72) Inventor: BOBBERT, Ilja, 1213 RT Hilversum (NL)
(74) Representative: van Heuvel, Margaretha
(86) International application number: PCT/EP2007/050306
(87) International publication number: WO 2007/080187

(56) References cited:
- WO-A-00/35289
- WO-A-97/25106
- WO-A-2004/067194
- WO-A-2006/076334
- DE-A1- 4 418 847
- FR-A- 2 751 634

## Description

The present invention relates to the field of disinfection and cleaning, more specifically to enhanced biocidal activity compositions based on hydrogen peroxide that also possess enhanced stability.

Numerous classes of chemical compounds exhibit varying degrees of biocidal or antimicrobial activity. Biocidal compositions are needed, among other things, to clean and disinfect food surfaces such as fruits and vegetables and to clean and disinfect hard-surfaces in the health care industry, food and beverage industries and household area.

In the past few years, efforts have been concentrated on developing chemicals that will be highly effective against microorganisms when in a diluted form, will be low in toxicity to humans and other animals, and will not be harmful to the environment.

Of the known disinfectants and biocidals, hydrogen peroxide appears to have exceptional potential, because the decomposition products, water and oxygen, are not toxic and not harmful to the environment. Also, it tends to have a broad spectrum biocidal activity. Broad spectrum activity is important for instance in situations where harmful organisms are present but.their identity is not known. Hydrogen peroxide-based disinfectants are useful in many different applications, including in hospitals, clinics, laboratories, dental offices, home care and chronic care facilities. They may also be used in food and beverage processing and preparation, animal husbandry, the hospitality industry and for general sanitation.

In order to provide fast, effective action, biocidal hydrogen peroxide solutions had to employ relatively high concentrations of hydrogen peroxide. However, at higher concentrations, the solutions may be subject to hazardous goods regulations and may require special precautions for handling and use. For example, at concentrations of above about 8 w/w% aqueous solution, hydrogen peroxide is considered corrosive and is also a strong oxidizing agent. Solutions containing less than about 8 w/w% hydrogen peroxide are preferred for their improved safety profile.

Compositions based on hydrogen peroxide as the only biocidal compound and containing up to 7% hydrogen peroxide by weight of the total composition are not fully efficacious to disinfect soiled surfaces, e.g., surfaces which needs both to be washed and disinfected. Indeed, the presence of organic and/or inorganic soils decreases the bactericidal activity of many antimicrobials like peroxygen-based agents, resulting thereby in a lower bactericidal activity and disinfection power of compositions comprising them.

At low concentrations (e.g. 3% w/w), hydrogen peroxide is non-irritating to skin, but exhibits low germicidal activity. For example, a solution containing 3% w/w hydrogen peroxide takes 20 minutes to achieve a greater than 6 log reduction in Staphylococcus aureus, which is too long for many applications. Increasing the concentration of hydrogen peroxide will increase the rate of disinfection. For example, a 25% w/w aqueous solution of hydrogen peroxide requires only 20 seconds to achieve a greater than log 6 reduction in Staphylococcus aureus. However, the solution is corrosive at this concentration and requires special handling procedures.

Another drawback of the use of hydrogen peroxide compositions is that without the use of a stabiliser, or a combination of stabilisers, the aqueous peroxide compositions characteristically may decompose over a relatively short time period.

Several solutions are proposed in the art to obtain hydrogen peroxide compositions with enhanced biocidal activity.

WO 97/31093 discloses a disinfecting composition comprising a peroxygen bleach, e.g. hydrogen peroxide, an amphoteric surfactant, e.g. betaine, glutaraldehyde and an antimicrobial essential oil.

WO 01/65939 discloses bactericidal properties of a combination of hydrogen peroxide, a benzalkonium salt and an inorganic phosphate sequestering agent.

US 6,479,454 and US 6,444,230 disclose improved antimicrobial activity of the combination of a peroxygen compound with an amine oxide.

WO 03/067989 and WO 00/35289 disclose the use of certain anionic sulfonic acid-based surfactants in combination with hydrogen peroxide.

It is an object of the present invention to provide compositions which deliver excellent biocidal activity using as low as possible hydrogen peroxide concentrations and/or as less as possible further biocidal additives. Also, an objective has been to provide a composition that can be applied without handling or usage precautions, safety measures, and that does not require rinsing or only scarce rinsing after application. It is surprisingly shown by the present invention that compositions comprising a combination of hydrogen peroxide with certain phosphate or phosphonate compounds show an enhanced biocidal activity. The compositions also exhibit a significant increase in stability as compared to hydrogen peroxide solutions not containing the compound in question.

WO 2004/067194 relates to stabilised aqueous compositions that contain hydrogen peroxide, an ethoxylated aliphatic phospono surfactant of formula (I), (HO)₍₃₋ₘ)OP(R-(CH₂-CH₂-O)ₙ-R¹)ₘ or (HO)₍₃₋ₘ₎OP(O-(CH₂-CH₂-O)ₙ-R¹)ₘ or (HO)₍₃₋ₘ)OP(O-R¹-(CH₂-CH₂-0)ₙ-H)ₘ, and at least one additional detersive surfactant. It was found that the compound of formula (I) provides the composition with very good aesthetic properties (a transparent gel) and helps stabilise the formula even at high temperature (around 40 °C for up to 6 months). In addition, the composition is stable to light, in particular UV light.

WO 97/47718 discloses a thickening agent for aqueous hydrogen peroxide solutions, ensuring a reliable and permanent viscosity setting and making the inclusion of perfumes easy without the risk of turbidity. The thickening agent comprises 10 to 90% fatty acid alkanol amide, 5 to 20% alkyl ether sulphate, and, optionally, up to 5% alkyl ether phosphate and up to 60% alkylpolyglycol ether.

However, no mentioning is made in these two documents of enhanced biocidal activity of compositions comprising ethoxylated aliphatic phospono surfactants / alkyl ether phosphates. In addition, compounds with a structure according to Formula 2 are not explicitly disclosed in these documents.

Thus, in a first aspect, the present invention provides the use as a biocidal composition of a composition comprising hydrogen peroxide in a concentration of 0.05-50% (w/w) and a compound with a structure according to Formula 1:

(OH)₍₂₋ₘ₎(X) (O)P-[(O)ₚ- (R')_{q}-(CH(Y)-CH₂-O)ₙ-R]ₘ, or a salt thereof,

wherein X is H or OH; each Y is independently H or CH₃; m is 1 and/or 2; each p and q are independently 0 or 1, with the proviso that when p is 0, q is 1; each n is independently 2-10; each R' is independently an alkylene radical containing 1-18 carbon atoms; each R is independently H or an alkyl radical containing 1-18 carbon atoms; and R'+R ≤ 20;
in a concentration of 0.01-60% (w/w).

The composition surprisingly has an excellent biocidal activity, even upon dilution to a composition comprising 0.0.5-8% of hydrogen peroxide and 0.01-10% of a compound with a structure according to Formula 1. It also displays a good stability over time. The combination of hydrogen peroxide and the compound according formula 1 provides a more potent biocidal composition than compositions that can be obtained by using these two compounds separately.

Unless indicated otherwise, percentages used throughout this invention are weight percentages based on the total weight of the composition.

Biocidal activity as referred to throughout this invention includes biocidal activity against all types of microorganisms, bacteria, yeasts and fungi, and against viruses.

Formula 1 relates to both individual, homogeneous compounds as well as heterogeneous mixtures of compounds. For instance, heterogeneous mixtures may contain compounds wherein the value for n and/or the length of the alkylene and/or alkyl radical ranges between different values, with the values for n and the chain lengths of R' and R as specified in this invention being average values. In addition, heterogeneous mixtures may contain a mixture of mono- and di-esters according to Formula 1. Mono- or di-esters according to Formula 1 are compounds wherein m is 1 or 2, respectively. It is preferred that in such mixtures the monoester is the predominant species, i.e. constitutes at least 50% of such a mixture, preferably at least 60%, more preferably at least 70%, even more preferably at least 80%, most preferably at least 90%.

In a preferred structure according to Formula 1, X is OH, Y is H, n is 2-8, preferably 2-6, more preferably 4-6, most preferably 4, and/or R'+R is 4-18 carbon atoms, preferably 4-16 carbon atoms, more preferably 6-14 carbon atoms, even more preferably 8-12 carbon atoms, even more preferably 8-10 carbon atoms. Also preferably, R and R' are straight chain radicals. In a further preferred structure, the value for p is 1 and for q is 0.

An especially preferred compound for use according to the invention forms another aspect of this invention. It is a compound with a structure according to Formula 1 wherein m is 1, p is 1 and q is 0, i.e. a compound with a structure according to Formula 2:

(OH)X)(O)P-O-(CH(Y)-CH₂-O)ₙ-R,

or a salt thereof,
wherein X is H or OH, Y is H or CH₃, n is 4-8 and R is an alkyl radical containing 4-1 carbon atoms, in a concentration of 0.01-60% (w/w).

In a preferred structure according to Formula 2, X is OH, Y is H, n is 4-6, preferably n is 4, and R is an alkyl radical containing 6-12 carbon atoms, more preferably 8-12 carbon atoms, most preferably 8-10 carbon atoms. Also preferably, R is a straight chain alkyl radical.

In an especially preferred structure according to Formula 2, X is OH, Y is H, n is 4-6, preferably n is 4, and R is a straight chain alkyl radical containing 8-12 carbon atoms, preferably 8-10 carbon atoms.

The composition for use according to the invention preferably may be sold as a concentrate comprising hydrogen peroxide in a concentration that may range from about 10-50% and the compound with a structure according to Formula 1 in a concentration that may range from about 5-60%. Said concentrate may suitably be diluted to the effective concentration to be used for final application.

Upon dilution, the effective hydrogen peroxide concentration of the composition may be 0.05-8% (w/w), preferably 0.1-5%, more preferably 0.2-3%, most preferably 0.3-2%. Depending on the intended use of the composition, the hydrogen peroxide concentration may be in the higher range, e.g. from 1-8%, or in the lower range, e.g. from 0.05-1%. The concentration of the compound with a structure according to Formula 1 may be 0.01-10% (w/w), preferably 0.05-5%, more preferably 0.1-2%.

The concentration of hydrogen peroxide and the compound with a structure according to Formula 1 in the composition preferably is chosen in such a way that the weight ratio between hydrogen peroxide and the compound with a structure according to Formula 1 varies between 10 and 0.1, more preferably between 5 and 0.2, most preferably between 2 and 0.5.

In one embodiment of the invention, the composition of the invention is formed by dissolving a dry particulate formulation in water. In this embodiment, hydrogen peroxide is generated from a peroxy compound such as sodium percarbonate, sodium perborate monohydrate, sodium perborate tetrahydrate, sodium tetraborate decahydrate, or mixtures thereof. This enables the use of a solid composition comprising the peroxide-generating compound and the compound with a structure according to Formula 1 or 2.

Due to the effectiveness of the combination of hydrogen peroxide and the compound with a structure according to Formula 1, the composition may be used as a formulation which is as simple as possible. For many applications it may not be necessary to supplement the composition with additional compounds influencing (enhancing) its biocidal activity: Thus, in such embodiments, the composition consists essentially of hydrogen peroxide and the compound with a structure according to Formula 1 as compounds with biocidal activity.

Biocidal activity of a composition for use according to the invention is preferably determined by a controlled bactericidal suspension test conform European Norm for chemical disinfectants and antiseptics EN 1276 (EN 1276: Quantitative suspension test for the evaluation of bactericidal activity of chemical disinfectants and antiseptics used in food, industrial, domestic, and institutional areas: test method and requirements). An effective biocidal composition is a composition that provides at least a log 3 reduction after a 1 minute contact time with a Staphylococcus aureus test suspension of 10⁸ cfu per ml, preferably at least a log 4 reduction, more preferably at least a log 5 reduction, most preferably at least a log 6 reduction.

Depending on the microorganism to be tested, it is possible to use suspension tests dedicated for use with fungi, yeasts or viruses. For fungi or yeasts, the tests according to European norms EN 1275, EN 1650 or EN 13624 may be used. For viruses, the test according to European norm EN 14476 may be used.

Finally, a test according to European norm EN 13704 is suitable for the evaluation of sporicidal activity of chemical disinfectants used in food, industrial, domestic and institutional areas.

The biocidal peroxide composition for use according to the invention preferably is an aqueous solution.

Examples of compounds with a structure according to Formula 1 are:

| Uniqema: | | |
|---|---|---|
| ■ | Monafax 1214 | aliphatic C8-10, 4EO |
| ■ | Monafax 831 | aliphatic |

| Basf: | | |
|---|---|---|
| ■ | Maphos 60A | aliphatic C10 ethoxylate - mono-ester |
| ■ | Maphos 58 | aliphatic |

| Akzo: | | |
|---|---|---|
| ■ | Phospholan PE 169 | C13 isotridecyl ether phosphate |

| Zschimmer & Schwarz: | | |
|---|---|---|
| ■ | Phosfetal 201 | Aliphatic C12 (mixture mono- en di-ester) |
| ■ | Phosfetal 213 | Aliphatic C18 (mixture mono- en di-ester) |
| Elementis: | | |
| ■ | SERVOXYL VPBZ 5/100, a C12-C16 coco-based 5 EO, mono- and di-ester. | |
| ■ | SERVOXYL VPDZ 3/100, a C13, 3 EO, mono- and di-ester (isotridecylalcohol (C13) polyethyleneglycol ether (3 EO) phosphate ester) | |
| ■ | SERVOXYL VPDZ 6/100, a C13, 6 EO, mono- and di-ester (isotridecylalcohol (C13) polyethyleneglycol ether (6 EO) phosphate ester) | |
| ■ | SERVOXYL VMDZ 6/100, a C13, 6 EO, ca. 90% mono-ester | |
| ■ | SERVOXYL VPTZ 3/100 a C8, 3 ethylene oxide, mono- and di-ester (2-Ethylhexanol polyethyleneglycol ether (3 EO) phosphate ester) | |

In a preferred embodiment, the biocidal peroxide composition for use according to the invention is a ready-to-use aqueous solution comprising 0.1-5% hydrogen peroxide and 0.05-5% of a compound with a structure according to Formula 1. The pH of the solution preferably is 2-5.

Such composition also is very ecologically friendly.

Especially preferred compounds for use according to the invention are compounds such as Phosfetal® 201, SERVOXYL VMDZ 6/100, SERVOXYL VPBZ 5/100 Maphos® 60A (BASF) and Monafax® 1214 (Uniqema). Especially Monafax® 1214 is a highly biodegradable product with very good biocidal properties in combination with hydrogen peroxide, and has received ecolabelling from the Swedish Society for Nature Conservation. This enables the use of the solution in situations where environmentally friendly products are preferred.

Various other compounds may be added to the composition to enhance its practical utility.

For instance, a pH adjusting acid (organic or inorganic) or base or an appropriate buffer may suitably be added to provide the composition with a pH of choice. Preferably, the composition has a pH in the acidic region, more preferably a pH of 1-8, even more preferably a pH of 1.5-6, and most preferably a pH of 2-5.

The composition further may comprise a hydrogen peroxide stabilizer, preferably in the form of a cation sequestering agent, more preferably in a concentration of 0.01 to 20% (w/w). The cation sequestering agent may be chosen from ethylenediaminetetraacetic acid (EDTA), diethylenetriaminepentaacetic acid (DTPA), N-(hydroxyethyl)-ethylenediaminetriacetic acid (HEDTA), nitrilotriacetic acid (NTA), 2-hydroxyethyliminodiacetic acid (HEIDA), and salts thereof or from benzoic acid, aminobenzoic acid, citric acid, phosphoric acid, iminodisuccinic acid and polyaspartic acid. More preferably, the cation sequestering agent is a (colloidal) stannate, and even more preferably is chosen from acetanilide, trisodium ethylenediamine disuccinate, for instance OctaQuest E30 or A65 (Octel), phosphonic acid derivatives having 1 to 5 phosphonic acid groups, for instance a Dequest phosphonate (Solutia), 1-hydroxyethylidene-1,1-diphosphonic acid, amino tri(methylene phosphonic acid), diethylenetriamine-penta(methylene phosphonic acid), 2-hydroxy ethylimino bis(methylene phosphonic acid), and ethylene diamine tetra(methylene phosphonic acid).

The composition further may comprise a corrosion inhibitor, preferably, in a concentration of 0.01% to 20% w/w. Preferably, the corrosion inhibitor is chosen from 1,2,3 benzotriazole, sodium molybdate, sodium nitrite, sodium bisulfate, sodiummetabisulfate, chromates, borates, phosphates, polyphosphates, sodium benzoate, sodium gluconate and sodium silicate.

The composition may also comprise a hydrogen peroxide compatible surfactant. This surfactant may be an anionic, a cationic, a nonionic and/or an amphoteric surfactant, preferably a nonionic and/or amphoteric surfactant. The surfactant concentration may be from 0.005 to 40% w/w.

Exemplary hydrogen peroxide compatible nonionic surfactants are amine oxides, ethoxylated fatty alcohols and/or alkyl(poly)glycosides.

Preferred hydrogen peroxide compatible nonionic surfactants are amine oxides, like C8-C20 alkyl dimethyl amine oxides and/or C8-C20 alkyl dihydroxyethyl amine oxides, such as dimethyldecylamine oxide or dimethylcocoamine oxide.

Preferred hydrogen peroxide compatible amphoteric surfactants are betaines, like C8-C20 alkyl dimethyl betaines, C8-C20 alkyl amidopropyl dimethyl betaines, such as cocamidopropyl dimethyl betaine, and/or C8-C20 alkyl sulfobetaines.

It was surprisingly found by the present invention that a composition comprising a combination of hydrogen peroxide, a compound with a structure according to Formula 1 and an amine oxide and/or a betaine displays a very good biocidal activity, while also showing very good cleaning and degreasing capacity.

As well, the composition may comprise at least one C1 to C8 alcohol, preferably in a concentration of about 0.01 to about 10% w/w. The alcohol may be chosen from benzyl alcohol, ethanol, n-butanol, 1-propanol, isopropanol and glycols, such as ethylene glycol, propylene glycol and butylene glycol.

Other additives may be added to the biocidal peroxide composition in order to provide the composition with properties suitable for its use. Examples of such additives' are emulsifiers, solvents, hydrotropes, glycerol, fragrances, coloring chemicals, preservatives, anti-foam and corrosion inhibitors.

The present invention provides the use of the biocidal hydrogen peroxide composition for any purpose where disinfecting and/or sanitizing activity is required. The composition further advantageously can be used for purposes where, next to disinfecting activity, cleaning and/or bleaching and/or preservative activity is required. The use according to the invention comprises contacting a substrate suspected to be contaminated with a microorganism with the biocidal hydrogen peroxide composition. Due to the effectiveness of the composition, the contact time usually does not need to be longer than 1-5 minutes. The use includes use as a bactericidal and sterilizing rinsing and cleaning liquid, and as a disinfection, cleaning and sanitization agent, for instance a disinfecting soap.

In particular, the biocidal peroxide composition may be used for those applications where it is important to provide disinfecting and/or sanitizing activity, preferably combined with cleaning and/or bleaching and/or preservative activity, with the mildest agents possible, for instance domestic use, medical use, personal care, mouth care, food, clean rooms, etc. Also for applications where no or scarce rinsing after application is preferred, or where the solution may come into contact with food.

Since the biocidal peroxide composition is non-irritating, has no odors or volatile gasses, and is skin friendly, it is also optimal for situations where users do not wear any protective clothing, in cases where worker-safety has high priority or for personal application like wound disinfection or prevention of gingivitis.

The present invention also relates to the use of the biocidal peroxide composition in specific devices such as spray devices, e.g. spray bottles, aerosol cans, aerosol generation devices for room disinfection, and by application in the form of dipping.

A preferred use of the biocidal hydrogen peroxide composition relates to the use as skin disinfecting agent, preferably for hand disinfection.

In order to enhance the practical utility and effectiveness as skin disinfectant, various skin-conditioning agents may be added to the composition. The skin-conditioning agent may be chosen from glycerides, sorbitol, castor oil, (water soluble) silicons, allantoin, cationic polymers, lanolin and its derivatives and cetyl alcohol.

The composition may further comprise nonionic surfactants to improve wetting capacity and enhance drying of the hands. Also fat, oil and/or stain removers and degreasers, such as anionic, nonionic or amphoteric surfactants or alcohols, may be added for specific situations where fat and stain removal is required.

A problem with existing skin disinfection products, typically containing alcohols in high concentrations, iodines/iodophors, chlorhexidine gluconate (CHG), phenolic compounds, quaternary ammonium compounds or combinations thereof, is that they often sacrifice disinfectant activity for the sake of skin mildness or vice versa. For example, while raising the concentration of the active ingredient may lead to a higher level of disinfection, such higher concentration frequently leads to increased skin irritation.

The skin (hand) disinfectant compositions may advantageously replace such disinfectants that have been developed to achieve high levels of disinfection where such need exists.

The composition for use according to the invention is able to provide adequate levels of disinfection while not being irritating to the skin. The composition is non-irritating due to the low levels of hydrogen peroxide, mild surfactant package and low concentrations of other mild additives which may be employed as described above. The solution has broad-spectrum activity, the degree of which is unexpected given the germicidal activity of the individual ingredients. A synergy exists amongst the ingredients of the present inventive solution such that an effective disinfectant is provided that is suitable for use on skin.

In a preferred embodiment, the composition is provided as a disinfecting soap, wherein the use of anti-microbial chemicals such as Triclosan, Chlorhexidine, PCMX, etc, is obviated. The soap may be formulated as a gel soap, spray soap or foam soap. It may be used as soap for disinfecting and cleaning skin or hair (shampoo) in general, more specifically for the hands or face, or may be used as animal shampoo.

As an additional surfactant, to further increase the biocidal capacity of the soap, the soap may preferably contain a betaine; like an alkyl dimethyl betaine, alkyl amidopropyl dimethyl betaine, such as cocamidopropyl dimethyl betaine, and/or an alkyl sulfobetaine, and/or an amine oxide, like an alkyl dimethyl amine oxide and/or an alkyl dihydroxyethyl amine oxide, such as an alkyl dimethyl amine oxide wherein the alkyl group has 10-18 carbon atoms, such as dimethyldecylamine oxide, dimethylcocoamine oxide and/or dimethylmyristylamine oxide.

Another preferred use of the composition relates to the use in dentistry and as mouth rinse. Infection and inflammation control in the mouth and oral cavities is still an important area and until today dominated by chlorine-, alcohol- and phenol-based products. Many of these products have significant drawbacks and have a negative influence on living tissue. The compositions of the present invention can effectively replace such products.

In order to have an effective composition for dentistry and mouth rinse, various compounds may be added to the composition to enhance its anti-microbial efficacy, such as anti-microbial essential oils and zinc salts, i.e. zinc chloride, zinc oxide, zinc lactate, or compounds that enhance the practical utility such as glycols, alcohols, edible surfactants, flavors, fragrances, etc.

The present invention further relates to the use of the composition for disinfecting, and preferably also cleaning a substrate. This can be done by contacting the substrate with an effective amount of the biocidal composition. In addition to disinfecting, the composition is especially effective in the removal of stains and dirt, for certain substrates accompanied by odor removal. The substrate may be any surface, space, material, medical instrument or device, hospital equipment, surface of walls, ceilings and/or floors. For instance, the composition can be effectively used for carpet disinfection and cleaning. Preferably, the substrate is a substrate wherein the presence of (pathogenic) micro-organisms is suspected.

The composition further may be effectively used for food preservation, as rinsing liquid for meat, poultry and fish, as rinsing liquid in breweries and dairy production, for veterinary and cattle applications, such as prevention and treatment of mastitis, and for water treatment and water disinfection.

In a preferred embodiment, the composition is provided as a rinsing liquid for machinery or equipment. Examples of the latter are food processing equipment, cutting machines, nozzles, brewery installations, bakery installations, dairy installations, fruit- and vegetable processing units, juice and soft drink installations, etc. medical equipment and instruments, and household equipment.

In this embodiment, the composition should be low foaming. An additional nonionic and/or amphoteric surfactant that may be present in the composition thus should not be a too highly foaming surfactant. Amine oxides and/or betaines with an alkyl chain in the lower ranges, e.g. 8-10 carbon atoms, are preferred, for instance octyl- and/or decyldimethylamine oxide. To further achieve low-foaming properties, the composition in the form of a rinsing liquid may be supplemented with a defoamer, like a water soluble silicone, and/or a low-foaming non-ionic surfactant with defoaming properties, like an alkyl polyalkylene glycol ether, for instance selected from the Propetal series from Zschimmer & Schwarz.

### Examples

Bactericidal activity of the exemplified compositions was tested using a controlled bactericidal suspension test conform European Norm for chemical disinfectants and antiseptics EN 1276 (EN 1276: Quantitative suspension test for the evaluation of bactericidal activity of chemical disinfectants and antiseptics used in food, industrial, domestic, and institutional areas: test method and requirements). One ml of a test suspension containing about 10⁸ cfu of the test microorganism per ml is added to 8 ml of the composition to be tested, and 1 ml milli-Q water is added. In some experiments, a protein load was added to this suspension according to the EN 1276 procedures to simulate unclean practical conditions. To provide for a clean condition 0.3% Bovine Albumin was added and for a dirty condition 3% Bovine Albumin.

After 1, 2 and 5 minutes contact time, the amount of viable bacteria was determined.

In a similar way, fungicidal and virucidal activity was tested using tests dedicated for use with fungi of viruses. For fungi or yeasts, the tests according to European norms EN 1275, EN 1650 or EN 13624 were used. For viruses, the test according to European norm EN 14476 was used.

Biocidal activity of some compositions was tested using a bactericidal suspension test conform European Norm EN 12054 for chemical disinfectants and antiseptics, in particular products for hygienic and surgical handscrub and handwash. One ml of a test suspension containing at least 1 x 10⁸ cfu per ml bacteria is mixed with 9 ml of the composition to be tested. Firstly, the starting suspension is counted by diluting to countable levels. For the handrub test undiluted test suspensions are used. For the handwash test a hard water diluent is used.

### Example 1

Various compositions were tested for biocidal activity and compared to standard, commercially available H₂O₂ solutions without any additions except for the stabilizers. The compositions tested included an aliphatic phosphate ester with 8-10 carbon atoms and 4 moles of ethyleneoxide (EO), for example such as sold by Uniqema International under the tradename Monafax 1214. Also a hydrogen peroxide stabilizer was present in the form of Trisodium Ethylenediamine Disuccinate, available from Octel under the tradename OctaQuest (OQ). The pH of such solution ranges between 2 and 4.5.The test results are presented in Table 1 below. It appears that the addition of Monafax significantly enhances biocidal activity of the composition.

**Table 1**

| | test suspension | 1 min | 2 min | 5 min |
|---|---|---|---|---|
| **1.0% H₂O₂ + 0.02% OQ** | | | | |
| Salmonella typhimurium | 4.80E+08 | >1000 | >1000 | >1000 |
| Escherichia coli | 6.00E+07 | >1000 | >1000 | 1000 |
| Pseudomonas aeruginosa | 1.25E+08 | >1000 | >1000 | 172 |
| Staphylococcus aureus | 5.35E+08 | >1000 | >1000 | 560 |
| Enterobacter cloacae | 7.50E+08 | >1000 | >1000 | >1000 |
| | | | | |

| **1.5% H₂O₂+ 0.02% OQ** | | | | |
|---|---|---|---|---|
| Salmonella typhimurium | 4.80E+08 | >1000 | >1000 | 1000 |
| Escherichia coli | 6.00E+07 | >1000 | 1000 | 688 |
| Pseudomonas aeruginosa | 1.25E+08 | 46 | 13 | 0 |
| Staphylococcus aureus | 5.35E+08 | >1000 | 1000 | 678 |
| Enterobacter cloacae | 7.50E+08 | >1000 | >1000 | 864 |
| | | | | |

| **1.75% H₂O₂ + 0.02% OQ** | | | | |
|---|---|---|---|---|
| Salmonella typhimurium | 4.80E+08 | >1000 | >1000 | >1000 |
| Escherichia coli | 6.,00E+07 | >1000 | >1000 | 408 |
| Pseudomonas aeruginosa | 1.25E+08 | 670 | 210 | 48 |
| Staphylococcus aureus | 5.35E+08 | >1000 | 876 | 272 |
| Enterobacter cloacae | 7.50E+08 | >1000 | >1000 | 1000 |
| | | | | |

| **2% H₂O₂ + 0.02% OQ** | | | | |
|---|---|---|---|---|
| Salmonella typhimurium | 4.80E+08 | >1000 | >1000 | 1000 |
| Escherichia coli | 6.00E+07 | >1000 | 1000 | 576 |
| Pseudomonas aeruginosa | 1.25E+08 | 1000 | 650 | 2 |
| Staphylococcus aureus | 5.35E+08 | >1000 | 528 | 192 |
| Enterobacter cloacae | 7.50E+08 | >1000 | 1000 | 1000 |
| | | | | |

| **0.6% H₂O₂ + 0.7% Monafax 1214 + 0.02% OQ** | | | | |
|---|---|---|---|---|
| Salmonella typhimurium | 4.80E+08 | 0 | | |
| Escherichia coli | 6.00E+07 | 0 | | |
| Pseudomonas aeruginosa | 1.25E+08 | 0 | | |
| Staphylococcus aureus | 5.35E+08 | 0 | | |
| Enterobacter cloacae | 7.50E+08 | 0 | | |
| | | | | |

| **1.0% H₂O₂ + 1% Monafax 1214 + 0.02% OQ** | | | | |
|---|---|---|---|---|
| Salmonella typhimurium | 4.80E+08 | 0 | | |
| Escherichia coli . | 6.00E+07 | 0 | | |
| Pseudomonas aeruginosa | 1.25E+08 | 0 | | |
| Staphylococcus aureus | 5.35E+08 | 0 | | |
| Enterobacter cloacae | 7.50E+08 | 0 | | |
| | | | | |

| **1.0% H₂O₂ + 0.5% Monafax 1214 + 0.02% OQ** | | | | |
|---|---|---|---|---|
| Salmonella typhimurium | 4.80E+08 | 0 | | |
| Escherichia coli | 6.00E+07 | 0 | | |
| Pseudomonas aeruginosa | 1.25E+08 | 0 | | |
| Staphylococcus aureus | 5.35E+08 | 0 | | |
| Enterobacter cloacae | 7.50E+08 | 0 | | |
| | | | | |

| **1.5% H₂O₂ + 0.1% Monafax 1214 + 0.02% OQ** | | | | |
|---|---|---|---|---|
| Salmonella typhimurium | 1.00E+08 | 0 | | |
| Escherichia coli | 4.00E+07 | 0 | | |
| Staphylococcus aureus | 1.35E+08 | 0 | | |
| Enterobacter cloacae | 1.15E+08 | 0 | | |
| | | | | |

| **0.5% H₂O₂ + 0.2% Monafax 1214 + 0.02% OQ** | | | | |
|---|---|---|---|---|
| Salmonella typhimurium | 1.00E+08 | 0 | | |
| Escherichia coli | 4.00E+07 | 0 | | |
| Staphylococcus aureus | 1.35E+08 | 0 | | |
| Enterobacter cloacae | 1.15E+08 | 0 | | |
| | | | | |

| **0.5% H₂O₂ + 0.5% Monafax 1214 + 0.02% OQ** | | | | |
|---|---|---|---|---|
| Salmonella typhimurium | 1.00E+08 | | 0 | |
| Escherichia coli | 4.00E+07 | | 0 | |
| Staphylococcus aureus | 1.35E+08 | | 0 | |
| Enterobacter cloacae | 1.15E+08 | | 0 | |

Without Monafax, only a few bacteria types show greater than log 5 reduction within 5 minutes, which is the test norm according standard EN 1276. With Monafax, a log 6 or even log 7 reduction is reached after already 1 minute.

The composition comprising 1% H₂O₂ and 1% Monafax was able to kill the following organisms within 30 seconds: Pseudomonas aeruginosa, Escherichia coli, Staphylococcus aureus, Enterococcus hirae, Proteus Vulgaris, Staphylococcus epidermidis, Streptococcus pyogenes, Salmonella typhimurium, Shigella sonnei, Lysteria monocytogenes, Legionella pneumoniae, Campylobacter jejuni, Klebsiella pneumoniae, Enterococcus faecium, Proteus mirabilis, Saccharomyces cerevisiae.

### Example 2

The performance of Monafax 1214 was compared with that of two widely used nonionic surfactants, the ethoxylated fatty alcohol Dehydol LT7 (C12-18 with 7 EO; Cognis) in 0.6% and Arlasolve 200 of Uniqema, a high HLB nonionic surfactant (polyoxyethylene isohexadecylether) in 0.7%, and a pH of around 5 using the EN 1276 test. The test results are presented in Table 2 below. It appears that the biocidal activity of compositions containing Monafax is significantly better than that of compositions containing the ethoxylated fatty alcohol or the high HLB nonionic surfactant.

**Table 2**

| | test suspension | 1 min | 2 min | 5 min |
|---|---|---|---|---|
| **1.5% H₂O₂ + 0.1% Monafax 1214 + 0.02% OQ** | | | | |
| Escherichia coli | 4.00E+07 | 0 | | |
| Staphylococcus aureus | 1.35E+08 | 0 | | |
| | | | | |

| **1.5% H₂O₂ + 0.6% Dehydol LT7 + 0.02% OQ** | | | | |
|---|---|---|---|---|
| Escherichia coli | 1.30E+08 | >500 | 96 | 12 |
| Staphylococcus aureus | 1.74E+09 | 220 | 196 | 60 |
| | | | | |

| **1.5% H₂O₂ + 0.7% Arlasolve 200 + 0.02% OQ** | | | | |
|---|---|---|---|---|
| Escherichia coli | 1.30E+08 | >500 | >500 | 48 |
| Staphylococcus aureus | 1.74E+09 | 304 | 204 | 72 |

### Example 3

The performance of the solutions of the invention was compared to that of amine oxides, a group of surfactants known from the prior art (for example: US 6,479,454 (Ecolab) and US 6,444,230 (Chemoxal) to possess significant biocidal activity in a hydrogen peroxide solution, even at lower concentration ranges.

To simulate unclean practical conditions, a protein load was added according to the EN 1276 procedures to bacterial test suspensions. To provide for a clean condition 0.3% Bovine Albumin was added and for a dirty condition 3% Bovine Albumin. The performance of Monafax 1214 was compared with that of the N,N-Dimethyldecylamine-N-oxide (Barlox 10s of Lonza Ltd.). A H₂O₂ concentration of 1.2% was used and a 0.6% of the respective surfactants. The solution containing Monafax has a pH of around 2.3 and the solution containing Barlox 10s has a pH of around 5. The test results are presented in Table 3 below. It appears that the biocidal activity of compositions containing Monafax is significantly.

**Table 3**

| | test suspension | clean | | dirty | |
|---|---|---|---|---|---|
| | | 2 min | 5 min | 2 min | 5 min |
| **Barlox 10s (Lonza)** | | | | | |
| Enterococcus hirae | 3.80E+08 | 8 | 0 | 6 | 1 |
| Bacillus cereus | 2.20E+08 | >300 | >300 | >300 | >300 |
| Candida albicans | 3.00E+08 | 300 | 250 | >300 | >300 |
| Staphylococcus aureus | 5.50E+08 | >300 | >300 | >300 | >300 |
| Lysteria monocytogenes | 3.00E+08 | >300 | >300 | >300 | >300 |
| | | | | | |

| **Monafax 1214 (Uniqema)** | | | | | |
|---|---|---|---|---|---|
| Lysteria monocytogenes | 3.00E+08 | 3 | 0 | 0 | 0 |
| Enterococcus hirae | 2.00E+08 | 0 | 0 | 0 | 0 |
| Escherichia coli | 3.00E+08 | 0 | 0 | 0 | 0 |
| Pseudomonas aeruginosa | 1.00E+08 | 0 | 0 | 0 | 0 |
| Staphylococcus aureus | 5.50E+08 | 0 | 0 | 0 | 0 |
| Enterobacter cloacae | 3.00E+08 | 0 | 0 | 0 | 0 |

| | | | | | |
|---|---|---|---|---|---|
| >300 indicates a number of colonies that cannot be counted (overgrown) | | | | | |

### Example 4

It also appears that the solutions of the invention are significantly more stable than solutions only containing hydrogen peroxide and commercially available hydrogen peroxide stabilizers. The stability of a 0.6% hydrogen peroxide solution in the presence of commercially available stabilizers was tested. Test settings were 37 °C for 30 days. The test results are presented in Table 4 below. The H₂O₂ concentration was measured with potassium permanganate titration. The blank contains only H₂O₂ with a stabilizer as added by the manufacturer (Solvay Chemicals).

The addition of Monafax 1214 enhances the stability of the hydrogen peroxide solution, even in the presence of commercially available hydrogen peroxide stabilizers.

**Table 4**

| **30 days at 37 °C** | **Octaquest 0.02%** | **Acetanilide 0.05%** | **Dequest 0.20%** | **Blank** |
|---|---|---|---|---|
| without Monafax 1214 | -6.1% | -2.3% | -13.2% | -3.4% |
| with Monafax 1214 | -1.0% | 0.0% | 0.0% | -0.4% |

### Example 5

A solution containing 0.5% hydrogen peroxide, 0.5% aliphatic phosphate ester (Monafax 1214 of Uniqema International), 0.5% Acetanilide as hydrogen peroxide stabilizer and 0.6% C10 alcohol ethoxylate (8 moles EO) (Lutensol XL 80 by BASF) and Potassium Hydroxide till pH 4.5 was prepared and used as hand disinfection scrub agent.

| | |
|---|---|
| Hydrogen Peroxide | 0.5% |
| Monafax 1214 (Uniqema) | 0.5% |
| Lutensol XL 80 (BASF) | 0.6% |
| Acetanilide | 0.5% |
| Potassium Hydroxide | to pH 4.5 |
| Demineralised Water | up to 100% |

The solution was tested for its antimicrobial activity using EN 12054 test method at S. aureus, P. aeruginosa, E. hirae, and E. coli and showed more than log 3 reduction which is the norm for a hand antiseptic.

### Example 6

An antimicrobial hand soap is prepared containing the following ingredients:
1.5% Hydrogen Peroxide
1.5% Monafax 1214 (aliphatic phosphate ester)
2% Tego Betaine F 50 (alkyl amidopropyl betaine)
1% Natrulon H-10 (polyglycerol)

The composition is buffered with NaOH to pH 4.5

The biocidal activity (EN 1276 at 5 minutes, expressed as logarithmic reduction factors of colony forming units per ml) is as follows:

| | Clean | Dirty |
|---|---|---|
| E. coli ATCC 25922 | > 6.2 | > 6.2 |
| Pseudomonas aeruginosa ATCC 15442 | > 6.2 | > 6.2 |
| Enterococcus hirae ATCC 10541 | > 5.6 | > 5.6 |

### Example 7

A machine rinsing liquid is prepared containing the following ingredients:
0.4% Monafax 1214 (Uniqema)
0.3% Barlox 10s (Lonza Inc.)
0.5% Propetal 120 (Zschimmer & Schwarz)
0.05% Surfadone LP 100 (International Specialty Products)
0.2% Dequest 2010 (Solutia)

This liquid was tested according the EN 1276 norm and showed the following biocidal activities (expressed as logarithmic reductions in colony forming units per ml) :

| | Clean | Dirty |
|---|---|---|
| E. coli ATCC 25922 | > 5 | > 5 |
| Pseudomonas aeruginosa ATCC 15442 | > 5 | > 5 |
| Enterococcus hirae ATCC 10541 | > 5 | > 5 |

### Example 8

In the tested compositions as shown below, 1.5% hydrogen peroxide solution was supplemented with 0.8% of a series of commonly available compounds according formula 1. Table 5 shows the log reduction on Staphylococcus aureus,

**Table 5. Efficacy Data of various compounds according to Formula 1**

| **Compound** | | **Log reduction Staph. aureus** | |
|---|---|---|---|
| Manufacturer | Type | 1 min | 5 min |
| BASF | Maphos 60A | > 6 | > 6 |
| Zschimmer & Schwarz | Phosfetal 201 | 5.5 | > 6 |
| Elementis | VPDZ 6/100 | 3.0 | 5.5 |
| Elementis | VPBZ 5/100 | > 6 | > 6 |

### Example 9

A composition comprising 2% H₂O₂ and 1% Monafax 1214 was prepared and tested against-Vacciniavirus, Feline Calici Virus and Adenovirus according to the EN 14776 norm:
EN 14476 - Chemical disinfectants and antiseptics - Virucidal quantitative suspension test for chemical disinfectants and antiseptics used in human medicine - Test methods and requirements (phase 2/step 1).

Surprisingly, the composition containing Monafax displayed potent virucidal activity (Table 6), whereas standard commercially available H₂O₂, without added Monafax, resulted in a considerably lower log reduction (Table 7).

**Table 6**

| | 1 minute | 3 minutes |
|---|---|---|
| Vacciniavirus | log > 5.4 | |
| Feline Calici Virus | | log > 5.4 |
| Adenovirus | | log > 5.4 |

**Table 7**

| | | 3 minutes | 5 minutes | 10 minutes |
|---|---|---|---|---|
| Feline Calici Virus | 2% H2O2 | log 0.2 | log 0.9 | |
| Adenovirus | 2% H2O2 | | log 0.6 | log 0.6 |
| Adenovirus | 3% H2O2 | | log 0.5 | log 0.6 |

### Example 10

A solution comprising 7% H₂O₂ and 4% Monafax 1214 was prepared and further tested against fungi according to the European norms EN 1275, EN 1650 and EN 13624.

EN 1275 - Chemical disinfectants and antiseptics - Quantitative suspension test for the evaluation of basic fungicidal or basic yeasticidal activity of chemical disinfectants and antiseptics - Test method and reguirements (phase 1).

EN 1650 - Chemical disinfectants and antiseptics - Quantitative suspension test for the evaluation of fungicidal activity of chemical disinfectants and antiseptics used in food, industrial, domestic, and institutional areas - Test method and requirements (phase 2, step 1).

EN 13624 - Chemical disinfectants and antiseptics - Quantitative suspension test for the evaluation of fungicidal activity of chemical disinfectants for instruments used in the medical area - Test method and requirements (phase 2, step 1).

The test results are shown in Tables 8 and 9.

**Table 8. Test results for Candida albicans ATCC 12031 under simulated clean and dirty conditions with 5 and 15 minutes contact time**

| | 5 minutes | 15 minutes |
|---|---|---|
| EN 1275 | log 3.3 | log > 5.3 |
| EN 1650 / EN 13624 clean | log 3.2 | log > 5.3 |
| EN 1650 / EN 13624 dirty | log 2.5 | log > 5.3 |

**Table 9. Test results for Aspergillus niger ATCC 16404 under simulated clean and dirty conditions with 5 and 15 minutes contact time**

| | 5 minutes | 15 minutes |
|---|---|---|
| EN 1275 | log 4.5 | log > 5.4 |
| EN 1650 / EN 13624 clean | log > 5.4 | log > 5.4 |
| EN 1650 / EN 13624 dirty | log 4.4 | log > 5.4 |

## Claims

1. Use of a composition comprising hydrogen peroxide in a concentration of 0.05-50% (w/w) and a compound with a structure according to Formula 1:
(OH)₍₂₋ₘ₎(X)(O)P-[(O)p-(R')_{q}-(CH(Y)-CH₂-O)ₙ-R]ₘ,
or a salt thereof,
wherein X is H or OH; each Y is independently H or CH₃; m is 1 and/or 2; each p and q are independently 0 or 1, with the proviso that when p is 0, q is 1; each n is independently 2-10; each R' is independently an alkylene radical containing 1-18 carbon atoms; each R is independently H or an alkyl radical containing 1-18 carbon atoms; and R'+R ≤ 20;
in a concentration of 0.01-60% (w/w), as a biocidal composition.

2. Use according to claim 1, wherein the compound with a structure according to Formula 1 is a mixture of compounds wherein m is 1 and compounds wherein m is 2, the compounds wherein m is 1 constituting at least 50% of the mixture, preferably at least 60%, more preferably at least 70%, even more preferably at least 80%, most preferably at least 90%.

3. Use according to claim 1, wherein the compound with a structure according to Formula 1 is a compound wherein m is 1.

4. Use according to any one of the claims 1-3, wherein X is OH and Y is H.

5. Use according to any one of the claims 1-4, wherein n is 2-8, preferably 2-6, more preferably 4-6, most preferably 4.

6. Use according to any one of the claims 1-5, wherein R'+R is 4-18 carbon atoms, preferably 4-16 atoms, more preferably 6-14 carbon atoms, even more preferably 8-12 carbon atoms, even more preferably 8-10 carbon atoms.

7. Use according to any one of the preceding claims, wherein R' and R are straight chain radicals.

8. Use according to any one of claims 1-7, wherein p is 1 and q is 0.

9. Use according to any one of the preceding claims, wherein the hydrogen peroxide concentration is 0.05-10% (w/w), preferably 0.1-7%, more preferably 0.2-5%, most preferably 0.3-3%.

10. Use according to any one of the preceding claims, wherein the concentration of the compound with a structure according to Formula 1 is 0.01-10% (w/w), preferably 0.05-5%, most preferably 0.1-2%.

11. Use according to any one of the preceding claims, wherein the concentration of hydrogen peroxide and the compound with a structure according to Formula 1 is chosen in such a way that the weight ratio between hydrogen peroxide and the compound with a structure according to Formula 1 varies between 10 and 0.1, preferably between 5 and 0.2, more preferably between 2 and 0.5.

12. Use according to any one of the preceding claims, wherein the composition has a pH of 0-9, preferably 1.5-7, more preferably 2-5.

13. Use according to any of the preceding claims for any purpose where disinfecting and/or sanitizing activity, preferably combined with cleaning and/or bleaching and/or preservative activity, is required.

14. A composition comprising hydrogen peroxide in a concentration of 0.05-50% (w/w) and a compound with a structure according to Formula 2:
(OH) (X) (O)P-O-(CH(Y)-CH₂-O)ₙ-R,
or a salt thereof,
wherein X is H or OH, Y is H or CH₃, n is 4-8, and R is an alkyl radical containing 4-14 carbon atoms, in a concentration of 0.01-60% (w/w).

15. The composition of claim 14, wherein X is OH, Y is H, n is 4-6, and R is an alkyl radical containing 6-12 carbon atoms, preferably 8-10 carbon atoms.

16. The composition of any one of claims 14 or 15, wherein R is a straight chain alkyl radical.

17. Use of the composition of any one of claims 14-16 for any purpose where disinfecting and/or sanitizing activity, preferably combined with cleaning and/or bleaching and/or preservative activity, is required.

## Patentansprüche

1. Verwendung einer Zusammensetzung umfassend Wasserstoffperoxid in einer Konzentration von 0,05-50% (w/w) und einer Verbindung mit der Struktur gemäß Formel 1:
(OH)₍₂₋ₘ)(X)(O)P-[(O)ₚ-(R')_{q}-(CH(Y)-CH₂-O)ₙ-R]ₘ,
oder eines Salzes davon,
wobei X H oder OH ist; jedes Y unabhängig H oder CH₃ ist; m 1 und/oder 2 ist; jedes p und q unabhängig 0 oder 1 sind, mit der Maßgabe dass, falls p 0 ist, q 0 ist; jedes n unabhängig 2-10 ist; jedes R' unabhängig ein Alkylenradikal ist, das 1-18 Kohlenstoffatome enthält; jedes R unabhängig H oder ein 1-18 Kohlenstoffatome umfassendes Alkylradikal ist; und R'+R ≤ 20;
in einer Konzentration von 0,01-60% (w/w), als eine biozide Zusammensetzung.

2. Verwendung nach Anspruch 1, wobei die Verbindung mit einer Struktur nach Formel 1 ein Gemisch von Verbindungen ist, in denen m 1 ist, und Verbindungen, in denen m 2 ist, wobei die Verbindungen, in denen m 1 ist, mindestens 50% des Gemischs, bevorzugt mindestens 60%, bevorzugter mindestens 70%, noch bevorzugter 80%, am bevorzugtesten 90 % ausmachen.

3. Verwendung nach Anspruch 1, wobei die Verbindung mit einer Struktur nach Formel 1 eine Verbindung ist, in der m 1 ist.

4. Verwendung nach einem der Ansprüche 1-3, wobei X OH ist und Y H ist.

5. Verwendung nach einem der Ansprüche 1-4, wobei n 2-8, bevorzugt 2-6, bevorzugter 4-6, am bevorzugtesten 4 ist.

6. Verwendung nach einem der Ansprüche 1-5, wobei R'+R 4-18 Kohlenstoffatome, bevorzugt 4-16 Atome, bevorzugter 6-14 Kohlenstoffatome, noch bevorzugter 8-12 Kohlenstoffatome, am bevorzugtesten 8-10 Kohlenstoffatome sind.

7. Verwendung nach einem der vorhergehenden Ansprüche, wobei R' und R geradkettige Radikale sind.

8. Verwendung nach einem der Ansprüche 1-7, wobei p 1 ist und q 0 ist.

9. Verwendung nach einer der vorhergehenden Ansprüche, wobei die Wasserstoffperoxidkonzentration 0,05-10% (w/w), bevorzugt 0,1-7%, bevorzugter 0,2-5%, am bevorzugtesten 0,3-3% ist.

10. Verwendung nach einem der vorherstehenden Ansprüche, wobei die Konzentration der Verbindung mit der Struktur nach Formel 1 0,01-10% (w/w), bevorzugt 0,05-5%, am bevorzugtesten 0,1-2% ist.

11. Verwendung nach einem der vorstehenden Ansprüche, wobei die Konzentrationen des Wasserstoffperoxids und der Verbindung mit einer Struktur nach Formel 1 so gewählt sind, dass das Gewichtsverhältnis zwischen Wasserstoffperoxid und einer Verbindung mit einer Struktur nach Formel 1 zwischen 10 und 0,1, bevorzugt zwischen 5 und 0,2, besonders bevorzugt zwischen 2 und 0,5 variiert.

12. Verwendung nach einer der vorherstehenden Ansprüche, wobei die Zusammensetzung einen pH von 0-9, bevorzugt 1,5-7, besonders bevorzugt 2-5 aufweist.

13. Verwendung nach einem der vorhergehenden Ansprüche für jeden Zweck, bei dem Entkeimungs- und/oder Desinfektionsaktivitäten, bevorzugt verbunden mit Reinigungs- und/oder Bleichungs- und/oder Konservierungsaktivitäten, erforderlich sind.

14. Eine Zusammensetzung umfassend Wasserstoffperoxid in einer Konzentration von 0,05-50% (w/w) und einer Verbindung mit einer Struktur nach Formel 2:
(OH)(X)(O)P-O(CH(Y)-CH₂-O)ₙ-R,
oder eines Salzes davon, wobei X H
oder OH ist, Y H oder CH₃ ist, n 4-8 ist, und R ein Alkylradikal ist, das 4-14 Kohlenstoffatome umfasst, in einer Konzentration von 0,01-60% (w/w).

15. Die Zusammensetzung nach Anspruch 14, wobei X OH ist, Y H ist, n 4-6 ist, und R ein Alkylradikal ist, das 6-12 Kohlenstoffatome, bevorzugt 8-10 Kohlenstoffatome umfasst.

16. Die Zusammensetzung nach einem der Ansprüche 14 oder 15, wobei R ein geradkettiges Radikal ist.

17. Verwendung nach einem der Ansprüche 14-16 für jeden Zweck, bei dem Entkeimungs- und/oder Desinfektionsaktivität, bevorzugt verbunden mit Reinigungs- und/oder Bleichungs- und/oder Konservierungsaktivität, erforderlich sind.

## Revendications

1. Utilisation d'une composition comprenant du peroxyde d'hydrogène dans une concentration de 0,05 à 50 % (p/p) et un composé ayant une structure répondant à la Formule 1 :
(OH)₍₂₋ₘ₎(X)(O)P-[(O)ₚ-(R')_{q}-(CH(Y)-CH₂-O)ₙ-R]ₘ,
ou un sel de celui-ci,
dans laquelle X représente un atome d'H ou un groupe OH ;chaque radical Y représente indépendamment un atome d'H ou un groupe CH₃ ; m vaut 1 et/ou 2 ;chaque nombre p et q vaut indépendamment 0 ou 1, à condition que lorsque p vaut 0, q vaut 1 ;chaque nombre n vaut indépendamment 2 à 10 ;chaque radical R' représente indépendamment un radical alkylène contenant de 1 à 18 atomes de carbone ;chaque radical R représente indépendamment un atome d'H ou un radical alkyle contenant de 1 à 18 atomes de carbone ; et R'+R ≤ 20 ;
dans une concentration de 0,01 à 60 % (p/p), en tant que composition biocide.

2. Utilisation selon la revendication 1, dans laquelle le composé ayant une structure répondant à la Formule 1 est un mélange de composés dans lesquels m vaut 1 et de composés dans lesquels m vaut 2, les composés dans lesquels m vaut 1constituantau moins 50 % du mélange, de préférence au moins 60 %, plus préférablement au moins 70 %, encore plus préférablement au moins 80 %, de manière préférée entre toutes au moins 90 %.

3. Utilisation selon la revendication 1, dans laquelle le composé ayant une structure répondant à la Formule 1 est un composé dans lequel m vaut 1.

4. Utilisation selon l'une quelconque des revendications 1 à 3, dans laquelle X représente un groupe OH et Y représente un atome d'H.

5. Utilisation selon l'une quelconque des revendications 1 à 4, dans laquelle n vaut de 2 à 8, de préférence de 2 à 6, plus préférablement de 4 à 6, de manière préférée entre toutes 4.

6. Utilisation selon l'une quelconque des revendications 1 à 5, dans laquelle R' + R représente de 4 à 18 atomes de carbone, de préférencede 4 à 16 atomes, plus préférablementde 6 à 14 atomes de carbone, encore plus préférablementde 8 à 12 atomes de carbone, encore plus préférablementde 8 à 10 atomes de carbone.

7. Utilisation selon l'une quelconque des revendications précédentes, dans laquelle R' et R sont des radicaux à chaîne linéaire.

8. Utilisation selon l'une quelconque des revendications 1 à 7, dans laquelle p vaut 1 et q vaut 0.

9. Utilisation selon l'une quelconque des revendications précédentes, dans laquelle la concentration du peroxyde d'hydrogène est de 0,05 à 10 % (p/p), de préférence de 0,1 à 7 %, plus préférablement de 0,2 à 5 %, de manière préférée entre toutes de 0,3 à 3 %.

10. Utilisation selon l'une quelconque des revendications précédentes, dans laquelle la concentration du composé ayant une structure répondant à la Formule 1 est de 0,01 à 10 % (p/p), de préférence de 0,05 à 5 %, de manière préférée entre toutesde 0,1 à 2 %.

11. Utilisation selon l'une quelconque des revendications précédentes, dans laquelle la concentration du peroxyde d'hydrogène et du composé ayant une structure répondant à la Formule 1 est choisie de manière à ce que le rapport pondéral entre le peroxyde d'hydrogène et le composé ayant une structure répondant à la Formule 1 varie entre 10 et 0,1, de préférence entre 5 et 0,2, plus préférablement entre 2 et 0,5.

12. Utilisation selon l'une quelconque des revendications précédentes, dans laquelle la composition a un pH de 0 à 9, de préférence de 1,5 à 7, plus préférablement de 2 à 5.

13. Utilisation selon l'une quelconque des revendications précédentes pour tout usage dans lequel une activité désinfectante et/ou assainissante, de préférence combinée à une activité de nettoyage et/ou de blanchiment et/ou de conservation, est nécessaire.

14. Composition comprenant du peroxyde d'hydrogène dans une concentration de 0,05 à 50 % (p/p) et un composé ayant une structure répondant à la Formule 2 :
(OH)(X)(O)P-O-(CH(Y)-CH₂-O)ₙ-R,
ou un sel de celui-ci,
dans laquelle X représente un atome d'H ou un groupe OH, Y représente un atome d'H ou un groupe CH₃, n vaut de 4 à 8, et R représente un radical alkyle contenant de 4 à 14 atomes de carbone, dans une concentration de 0,01 à 60 % (p/p).

15. Composition selon la revendication 14, dans laquelle X représente un groupe OH, Y représente un atome d'H, n vaut de 4 à 6, et R représente un radical alkyle contenant de 6 à 12 atomes de carbone, de préférence de 8 à 10 atomes de carbone.

16. Composition selon l'une quelconque des revendications 14 ou 15, dans laquelle R représente un radical alkyle à chaîne linéaire.

17. Utilisation de la composition selon l'une quelconque des revendications 14 à 16 pour tout usage dans lequel une activité désinfectante et/ou assainissante, de préférence combinée à une activité de nettoyage et/ou de blanchiment et/ou de conservation, est nécessaire.
